# EUROPEAN PATENT SPECIFICATION

(11) **EP 2 162 141 B1**
(45) Date of publication and mention of the grant of the patent: **11.09.2013**
(21) Application number: 08753410.3
(22) Date of filing: 09.05.2008
(51) Int. Cl.: A61K 35/28, A01N 63/00, A61K 45/06, A61P 25/02, A61K 9/10, A61K 38/38, A61K 9/08, A61K 31/10, A61K 31/198, A61K 47/02, A61K 47/18, A61K 47/20, A61K 47/26, A61K 47/42, A61K 9/00

(54) **COMPOSITION FOR TREATING ISCHEMIC LIMB DISEASE COMPRISING STEM CELLS DERIVED FROM ADIPOSE TISSUE**
ZUSAMMENSETZUNG ZUR BEHANDLUNG VON ISCHÄMISCHER ERKRANKUNG DER EXTREMITÄTEN MIT STAMMZELLEN AUS FETTGEWEBE
COMPOSITION POUR LE TRAITEMENT D'UNE MALADIE ISCHÉMIQUE DES MEMBRES COMPRENANT DES CELLULES SOUCHES DÉRIVÉES DE TISSU ADIPEUX

(30) Priority: 25.05.2007 KR 20070050624
(43) Date of publication of application: 17.03.2010
(73) Proprietor: RNL Bio Co., Ltd., Gwanak-gu Seoul 151-050 (KR)
(72) Inventor: RA, Jeong Chan, Gyeonggi-do 440-300 (KR); HAN, Hae Jung, Yongin-si Gyeonggi-do 446-718 (KR); LEE, Hang Young, Suwon-si Gyeonggi-do 440-719 (KR); KIM, Hyo Eun, Seoul 152-770 (KR)
(74) Representative: Hiebl, Inge Elisabeth
(86) International application number: PCT/KR2008/002614
(87) International publication number: WO 2008/147057

(56) References cited:
- WO-A1-2006/134951
- WO-A2-03/059272
- WO-A2-2005/034843
- WO-A2-2006/136244
- THIRUMALA S ET AL: "Transport phenomena during freezing of adipose tissue derived adult stem cells", BIOTECHNOLOGY AND BIOENGINEERING 20051105 JOHN WILEY AND SONS INC. US, vol. 92, no. 3, 5 November 2005 (2005-11-05), pages 372-383, XP009142402, DOI: DOI:10.1002/BIT.20615
- REHMAN J. ET AL.: 'Angiogenic potential of subcutaneous adipose stromal cells for autologous cell therapy' JOURNAL OF THE AMERICAN COLLEGE OF CARDIOLOGY vol. 41, no. 6, SUPPL. A, 19 March 2003, page 308A, XP009028844
- PLANAT-BENARD V. ET AL.: 'Plasticity of Human Adipose Lineage Cells Toward Endothelial Cells: Physiological and Therapeutic Perspectives' CIRCULATION vol. 109, no. 5, 10 February 2004, pages 656 - 663, XP009034616
- MIRANVILLE A. ET AL.: 'Human adipose tissue-derived stem cells improve blood flow in the ischemic mouse hind-limb' CIRCULATION vol. 108, no. 17, SUPPL., 28 October 2003, page IV-164, XP008048883
- STASHOWER M. ET AL.: 'Stromal progenitor cells present within liposuction and reduction abdominoplasty fat for autologous transfer to aged skin' DERMATOLOGIC SURGERY vol. 25, no. 12, 1999, pages 945 - 949, XP002233641
- RANGAPPA S. ET AL.: 'Transformation of adult mesenchymal stem cells isolated from the fatty tissue into cardiomyocytes' ANNALS OF THORACIC SURGERY vol. 75, no. 3, March 2003, pages 775 - 779, XP002401034

## Description

### TECHNICAL FIELD

The present invention relates to cell therapeutic compositions, and more particularly to cell therapeutic compositions for use for treating ischemic diseases, wherein the compositions contain adipose tissue-derived mesenchymal stem cells and excipients.

### BACKGROUND ART

Ischemic limb diseases include diabetic foot, showing peripheral circulatory disturbance caused by occlusion of arteries or veins due to intravascular thrombosis, embolism, intravascular inflammation, hypertension, hyperlipidemia and diabetic complications, symptoms occurring in limbs due to blood vessel injury caused by external factors, such as bums or chilblains, Buerger's disease appearing as vascular inflammation and thrombosis in middle-aged men who are heavy smokers, typical examples of which are arteriosclerosis obliterans, thromboangiitis obliterans and the like.

Current methods for treating such diseases include preferentially treating diseases causing thrombosis or embolism, for example, arrhythmia, atrial myxoma and erythrocytosis, and temporarily relieving symptoms using thrombolytic agents or administering a vasodilator and a circulation-improving agent, etc. for incleasing the luminal diameter, for example, dextran, anticoagulants, phenylbutazone, persantin, adrenal cortex hormones, immunosuppressive agents or prostaglandin E1.

Surgical treatment methods include a method of removing pains in ischemic areas through sympathectomy, epidural blockade and sympathetic block, a method of relieving excessive sympathetic tone to induce the effect of improving vasodilation and blood circulation, endarterectomy, bypass graft surgery using artificial blood vessels such as Gortex, and lesionectomy.

However, the above-described drug treatment or surgical treatment methods have problems in that they temporarily relieve ischemic disease symptoms or show a high risk of recurrence. For this reason, there is a need to develop a method capable of fundamentally treating ischemic diseases.

Stem cells refer to cells having not only self-replicaiton ability but also the ability to differentiate into at least two cells, and can be divided into totipotent stem cells, pluripotent stem cells, and multipotent stem cells.

Recently, adipose tissue was found to be a new source of multipotent stem cells (Cousin et al., BBRC., 301:1016, 2003; Miranville et al., Circulation, 110:349, 2004; Gronthos et al., J Cell Physiol., 189:54, 2001; Seo et al., BBRC., 328:258, 2005). Namely, it was reported that a group of undifferentiated cells is included in human adipose tissue obtained by liposuction and has the ability to differentiate into fat cells, osteogenic cells, myoblasts and chondroblasts (Zuk et al., Tissue Eng., 7:211, 2001; Rodriguez et al., BBRC., 315:255, 2004). This adipose tissue has an advantage in that it can be extracted in large amounts, it can be proliferated in large amounts by tissue culture, and it can be repeatedly injected since it is autologous tissue so that there is no risk of immune rejection reaction. Therefore, it receives attention as a new source of stem cells, which overcomes the existing shortcomings of stem cells derived from bone marrow or umbilical cord blood, such as difficulties in collecting thereof, high cost, and having to find a suitable match through the tissue compatibility verification.

Also, recent studies using animal model experiments indicate that adipose tissue-derived cells have the abilities to regenerate muscles and to stimulate the differentiation of nerve blood vessels. Thus, these adipose tissue-derived cells have attention as a new source of stem cells.

Adipose tissue-derived stem cells known till now include human adipose-derived adult stem cells that can differentiate into epithelial cells (Brzoska et al., BBRC, 330:142, 2005), human adipose-derived adult stem cells that can differentiate into osteogenic and fat cells (Cao et al., BBRC, 332:370, 2005), human adipose-derived adult stem cells that can differentiate into nerve cells (Safford et al., BBRC, 294:371, 2002), rat adipose-derived stem cells that can differentiate into fat cells (Ogawa et al., BBRC, 319:511, 2004), rat adipose-derived stem cells that can differentiate into osteogenic and chondrogenic cells (Ogawa et al., BBRC, 313:871, 2004), human adipose-derived stem cells that can differentiate into cartilage cells (Biomaterials, 25:3211, 2004), rat adipose-derived stem cells that can differentiate into nerve cells (Fujimura et al., BBRC, 333:116, 2005), and adipose-derived stem cells that can differentiate into bone cells, cartilage cells, nerve cells or muscle cells (US 6,777,231).

Document WO 2006/134951 discloses a fat-derived stem cell isolated from a fat aspirate and a characterization of said stem cell. Further disclosed is a culture method for an undifferentiated stem cell or a cultured pluripotent stem cell, especially a long-term culture for a fat-derived stem cell. Further disclosed is the treatment of ischemia by administration of adipose-derived stem cells.

Document WO 2006/136244 discloses methods and compositions utilizing adipose-derived stromal cells for treating fistulae. Further disclosed are suitable carriers for adipose tissue derived stromal stem cells including sugars and polyols.

Document WO 03/059272 discloses a method for amplifying the production of angiogenesis and vasculogenesis inducing factors secreted by exposing stem cells to and co-culturing the stem cells with a compound for increasing the production of angiogenesis and vasculogenesis inducing factors. Further disclosed is a therapeutic for use in inducing angiogenesis and vasculogenesis, the therapeutic including angiogenesis and vasculogenesis inducing factors isolated from stem cells in conjunction with a pharamaceutically acceptable cell therapy.

In attempts to treat ischemic diseases, Korean Patent Publication No. 2003-0034177 discloses a method of treating ischemic diseases using hematopoietic stem cells whose differentiation has been induced by G-CSF, and Korean Patent Publication No. 2005-0111593 discloses a method of treating ischemic diseases using mesenchymal stem cells having an angiopoietin gene introduced therein. However, the prior methods have problems in that stem cells are used auxiliarily or in that the storage of cell therapeutic agents comprising stem cells is not stable.

Accordingly, the present inventors have made many efforts to develop a method of treating ischemic diseases using more stable stem cells and, as a result, have found that, when adipose stem cells derived from adipose tissue, to which an excipient such as sucrose has been added, are introduced into a mouse model of ischemic disease, new blood vessels are effectively produced, thereby completing the present invention.

### SUMMARY OF THE INVENTION

It is an object of the present invention to provide cell therapeutic composition s for use for treating ischemic diseases, which contain adipose tissue-derived mesenchymal stem cells as active ingredients and show high stability during cold or frozen storage.

To achieve the above object, the present invention provides a cell therapeutic composition, which contains 1 x 10⁵ - 1 x 10⁸ cells/mL adipose tissue-derived mesenchymal stem cells, saline, 2% sucrose, 5% albumin and 1 mM EDTA.

The invention further provides a cell therapeutic composition, which contains 1 x 10⁵ - 1 x 10⁸ cells/mL adipose tissue-derived mesenchymal stem cells, saline, 2% sucrose, 2% albumin and 10mM EDTA.

The invention further provides a cell therapeutic composition, which contains 1 x 10⁵ - 1 x 10⁸ cells/mL adipose tissue-derived mesenchymal stem cells, saline, 2% sucrose, 5% albumin and 10% DMSO.

The above cell therapeutic compositions according to the present invention are provided for use for treating ischemic diseases.

In the present invention, a base for the composition is preferably selected from the group consisting of physiological saline, phosphate buffered saline (PBS) and Hartman-D®.

In the present invention, the composition contains sucrose and albumin as the excipient. In addition, the composition contains EDTA or DMSO as the excipient.

In the present invention, the compositions preferably additionally comprise a pharmaceutically acceptable amount of at least one additive selected from the group consisting of suspending agents, solubilizing agents, stabilizers, isotonic agents, preservatives, adsorption-preventing agents, surfactants, diluents, vehicles, pH-adjusting agents, analgesic agents, buffering agents, sulfur-containing reducing agents and antioxidants.

Other features and embodiments of the present invention will become apparent from the following detailed description and the appended claims.

### BRIEF DESCRIPTION OF DRAWINGS

FIG. 1 shows photographs taken at 100x magnification for the human adipose tissue-derived multipotent stem cells according to the present invention.
FIG. 2 shows immunological characteristics of the human adipose-derived multipotent stem cells according to the present invention.
FIG. 3 shows stability of the human adipose tissue-derived multipotent stem cells according to the present invention in relation to sphere formation ability
FIG. 4 shows test results for the *in vitro* angiogenic ability of human adipose tissue-derived mesenchymal stem cells according to the present invention.
FIG. 5 shows the degree of limb removal in nude mice with an induced ischemic lower limb disease and injected with various concentrations of the inventive human adipose tissue-derived mesenchymal stem cells, and shows the degree of angiogenesis in the nude mice, observed using laser Doppler perfusion imaging.
FIG. 6 shows aniogenic ability of the inventive human adipose tissue-derived multipotent stem cells, expressed as laser Doppler perfusion ratio.

### DETAILED DESCRIPTION OF THE INVENTION, AND PREFERRED EMBODIMENTS

The present invention relates to cell therapeutic compositions and to their use for treating ischemic diseases, wherein the compositions contain adipose tissue-derived mesenchymal stem cells at a concentration of 1 x 10⁵-1 x 10⁸ cells/mL. and sucrose, albumin and EDTA or DMSO as excipients.

In the present invention, the adipose-derived stem cells are obtained from adipose tissue harvested through liposuction, and adipose-derived mesenchymal stem cells which adhere to a culture flask and grow *in vitro.*

In an embodiment of the present invention, the adipose tissue-derived mesenchymal cells can be obtained by infusing a tumescent solution and a fat-containing material using liposuction tubing or a disposable syringe having a catheter connected thereto, subjecting the resulting materials to a mycoplasma test and a sterility test, selecting a sample, which passed quality management standards, from among the tested materials, centrifuging the selected sample into an adipose layer and an aqueous layer, pre-treating the aqueous layer sample with a collagenase solution, and then culturing the resulting cells in a DMEM medium containing 10% FBS and ascorbic acid.

Meanwhile methods for obtaining multipotent stem cells expressing desired surface antigens from the human adipose tissue-derived stem cell broth obtained above include a FACS method using a flow cytometer with sorting function (Int. Immunol., 10(3):275, 1998), a method using magnetic beads, and a panning method using an antibody specifically recognizing multipotent stem cells (J. Immunol., 141(8):2797, 1998). Also, methods for obtaining multipotent stem cells from a large amount of culture broth include a method where antibodies, which are expressed on the surface of cells to specifically recognize molecules (hereinafter, referred to as "surface antigens"), are used alone or in combination as columns.

Flow cytometry sorting methods may include a water drop charge method and a cell capture method. In any of these methods, an antibody specifically recognizing an antigen on the cell surface is fluorescently labeled, the intensity of fluorescence emitted from an antibody bonded with the molecule expressed on the surface of the cell is converted to an electric signal whereby the expressed amount of the antigen can be quantified. It is also possible to separate cells expressing a plurality of surface antigens by combination of fluorescence types used therefor. Examples of fluorescences which can be used in this case include FITC (fluorescein isothiocyanate), PE (phycoerythrin), APC (allo-phycocyanin), TR (Texas Red), Cy 3, CyChrome, Red 613, Red 670, TRI-Color, Quantum Red, etc.

FACS methods using a flow cytometer include: a method where the above stem cell broth is collected, from which cells are isolated by, for example, centrifugation, and stained directly with antibodies; and a method where the cells are cultured and grown in a suitable medium and then stained with antibodies. The staining of cells is performed by mixing a primary antibody recognizing a surface antigen with a target cell sample and incubating the mixture on ice for 30 minutes to 1 hour. When the primary antibody is fluorescently labeled, the cells are isolated with a flow cytometer after washing. When the primary antibody is not fluorescently labeled, cells reacted with the primary antibody and a fluorescent labled secondary antibody having binding activity to the primary antibody are mixed after washing, and incubated on ice water for 30 minutes to 1 hour. After washing, the cells stained with the primary and secondary antibodies are isolated with a flow cytometer.

Also, the isolated mesenchymal stem cells according to the present invention can be analyzed for their immunological properties using flow cytometry.

As used herein, the term "ischemic diseases" refers to functional abnormality, tissue denaturation or necrosis, caused by the reduction or disruption of blood supply to tissues, and specifically includes ischemic heart diseases such as myocardial infarction and angina pectoris, extremity ischemia, injuries associated with impaired circulation, traumatic injuries such as amputation, and fractures. Specifically, the ischemic diseases in the present invention include not only ischemic diseases but also ischemic states resulting from injury or damage.

The cell therapeutic compositions of the present invention can be introduced directly or via a carrier. The carrier should be physiologically acceptable, and examples thereof include organic substances, such as biopolymers, and inorganic substances such as hydroxyapatite. Specific examples thereof include collagen matrices, polylactic acid polymers or copolymers, polyethylene glycol polymers or copolymers, and chemical derivatives thereof. Furthermore, the carrier may be a mixture of two or more of the above-described physiologically acceptable materials.

The compositions for use of the present invention can be administered, for example, at one or more sites (for example, 2-50 sites) in the surviving muscle (skeletal muscle, cardiac muscle, etc.) surrounding the ischemic site. The dosage of the composition is preferably in the range of, for example, 1.0 x 10⁵-1.0 x 10⁸ cells/kg (weight), and more preferably 1.0 x 10⁶-1.0 x 10⁷ cells/kg (weight).

However, the dosage of the compositions may vary depending on patient's weight, age, sex and symptoms, the dosage form of the composition to be administered, a method of administering the composition, and so on. Those skilled in the art can appropriately adjust the dosage. The frequency of administration may range from one to several times within clinically acceptable limits of side effects. There may be one or more administration sites. The dosage per kg for non-human animals may be the same as that for human, or can be converted from the above-described dosage, for example, based on the volume ratio (for example, average value) between the ischemic organs (such as heart) of the human and animal subjects. Animals to be treated according to the present invention include human and other desired mammals, specific examples of which include humans, monkeys, mice, rats, rabbits, sheep, cows and dogs.

The therapeutic use of the present invention can be conducted alone or in combination with other standard or advanced methods. For example, the inventive use for treating ischemic diseases can preferably be used in combination with surgical revascularization, such as percutaneous transluminal coronary angioplasty (PTCA) and/or coronary artery bypass graft (CABG). The use of the inventive use in combination with other methods can actively improve cardiac functions and reduce the period of being confined to bed.

The cell therapeutic compositions of the present invention may comprise pharmaceutically acceptable carriers and/or additives. Examples thereof include sterilized water, physiological saline, a standard buffer (e.g., phosphoric acid, citric acid, or other organic acids), a stabilizer, salt, an antioxidant (e.g., ascorbic acid), a surfactant, a suspending agent, an isotonic agent, or a preservative. For local administration, the compositions of the present invention are preferably combined with an organic substance such as biopolymer, an inorganic substance such as hydroxyapatite, specific examples of which include collagen matrix, a polymer or copolymer of polylactic acid, a polymer or copolymer of polyethylene glycol, and chemical derivatives thereof.

As used herein, the term "base" refers to a base solution in which the mesenchymal stem cells in the cell therapeutic composition are suspended. As the base, physiological saline, phosphate buffered saline or Hartman-D (Choongwae Pharma Corp.) is preferably used.

In a preferred embodiment, the cell therapeutic composition is prepared in a dosage form suitable for injection. For this purpose, the adipose stem cells of the present invention are preferably dissolved in a pharmaceutically acceptable aqueous solution, or frozen in a solution state. A kit comprising the composition(s) of the present invention may further comprise a desired pharmaceutically acceptable carrier that can be used to suspend or dilute the adipose stem cells. Examples of such a carrier include distilled water, physiological saline, PB S and the like.

The compositions of the present invention may contain a pharmaceutically acceptable carrier or excipient, or any necessary stabilizer or adsorption-preventing agent to provide a pharmaceutical preparation that is suitable for administration to humans or animals. The compositions of the present invention may be formulated in the form of an injectable solution (e.g., injection solutiuons for subcutaneous, intradermal, intramuscular, intravenous and intraperitoneal injection). Upon the injection of the inventive composition, an analgesic agent, which can relieve pains, may be used, and if necessary, a suitable device may also be used.

The cell therapeutic compositions of the present invention may be filled into a syringe, a device, a cryovial in which cells can be frozen, or a pyrogen-free glass vial comprising rubber stoppers and aluminum caps, which contains liquid drugs.

As the device, a syringe or a multi-syringe can be used, and in the case of limb ischemic diseases, a 20-30 gauge needle, which can minimize pain during the administration of cells without causing damage to cells due to the shearing of cells, is used considering the depth of a site or muscle into which cells are administered. Also, the syringe or device is made of a material which does not influence cell viability.

The cell therapeutic compositions of the present invention may, if necessary, contain at least one selected from among suspending agent, solubilizing agents, stabilizers, isotonic agents, preservatives, adsorption-preventing agents, surfactants, diluents, vehicles, pH-adjusting agents, analgesic agents, buffering agents, sulfur-containing reducing agents and antioxidants, depending on the administration mode or formulation thereof.

Examples of the suspending agents may include methylcellulose, Polysorbate 80, hydroxyethylcellulose, gum acacia, gum tragacanth powder, sodium carboxymethylcellulose, polyoxyethylene sorbitan monolaurate, etc. The solubilizing agents include polyoxyethylene hydrogenated castor oil, polysorbate 80, nicotinamide, polyoxyethylene sorbitan monolaurate, Macrogol and castor oil fatty acid ethyl esters. The stabilizers include dextran 40, methylcellulose, gelatin, sodium sulfite, sodium metasulfite, etc. Examples of the isotonic agents are D-mannitol and sorbitol.

Examples of the preservatives include methyl parahydroxybenzoate, ethyl parahydroxybenzoate, sorbic acid, phenol, cresol, and chlorocresol. Examples of the adsorption preventing agents include human serum albumin, lecithin, dextran, ethylene oxide-propylene oxide copolymer, hydroxypropylcellulose, methylcellulose, polyoxyethylene hydrogenated castor oil, and polyethylene glycol.

The sulfur-containing reducing agents include N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and sulfhydryl-containing compounds such as thioalkanoic acid having 1 to 7 carbon atoms.

The antioxidants include, for example, erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate. The cryopreservatives include, for example, DMSO, glycerol, etc.

Furthermore, the compositions of the present invention may comprise conventional additives, such as inorganic salts, including sodium chloride, potassium chloride, calcium chloride, sodium phosphate, potassium phosphate and sodium hydrogen carbonate, and organic salts, including sodium citrate, potassium citrate and sodium acetate.

Particularly, in the case of prior cell therapeutic agents, which were suspended and stored in physiological saline under cold-storage conditions, it was difficult to show a cell viability of more than 70%, when they were stored for 48 hours or more; however, in the case of the inventive cell therapeutic compositions containing human adipose tissue-derived mesenchymal stem cells, when sucrose or albumin was added to the composition, it showed a cell viability of more than 70%, even when it was stored for 48-72 hours, suggesting that it had improved stability. Also, in the case of the inventive compositions, it was observed that, even when Hartman-D or PBS was used instead of physiological saline as the base, there was no significant difference in cell stability, and the addition of sucrose or albumin improved the stability of the cell therapeutic compositions. Moreover, when the inventive cell therapeutic compositions containing adipose tissue-derived stem cells were stored under frozen storage conditions, the composition containing physiological saline, sucrose, albumin and cryopreservative DMSO showed the highest cell viability upon thawing, and it was observed that the addition of albumin and sugar components to the cell therapeutic compositions protected the cells during the freezing and thawing of the cells to improve the viability of the cells.

### Examples

Hereinafter, the present invention will be described in further detail with reference to examples.

### Example 1: Isolation of multipotent stem cells from adipose tissue

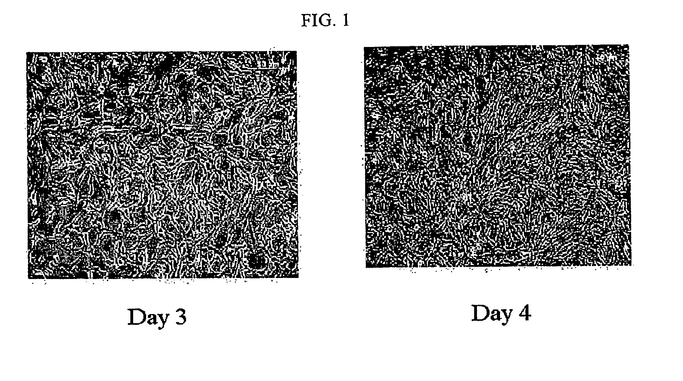

Adipose tissue was obtained from abdominal liposuction, and washed with PBS and then finely cut. The cut tissue was digested in DMEM media supplemented with collagenase type 1 (1mg/ml), at 37°C for 2 hours. The digested tissue was washed with PBS and then centrifuged at 1000 rpm for 5 minutes. The supernatant was suctioned off, and the pellets remaining on the bottom were washed with PBS and then centrifuged at 1000 rpm for 5 minutes. The resulting pellets were filtered through a 100 µm mesh to remove debris, followed by washing with PBS. The resulting cells were incubated in a DMEM medium (10% FBS, 2 mM NAC, 0.2 mM ascorbic acid). After one overnight period, unattached cells were washed with PBS, and cultured in Keratinocyte-SFM media (containing 2 mM NAC, 0.2 mM ascorbic acid, 0.09 mM calcium, 5 ng/ml rEGF, 50 µg/ml BPE, 5 µg/ml insulin and 74 ng/ml hydrocortisone) while the media were replaced at two-day intervals, thus isolating multipotent stem cells (FIG. 1). FIG. 1 shows photographs taken at 100x magnification for the human adipose tissue-derived multipotent stem cells isolated as described above.

### Example 2: Immunological characteristics of adipose-derived multipotent stem cells

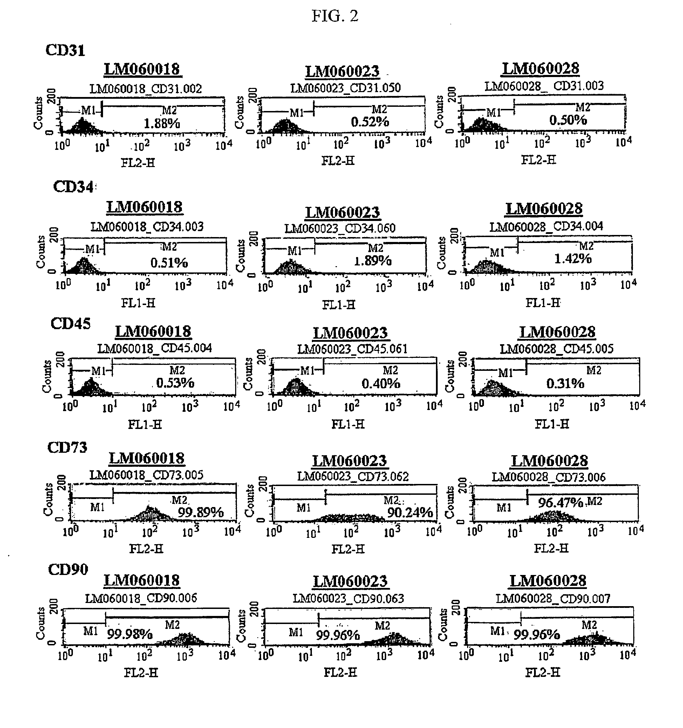

The adipose tissue-derived multipotent stem cells obtained in Example 1 were washed with PBS and treated with trypsin. The treated cells were collected and centrifuged at 1000 rpm for 5 minutes. The supernatant was discarded and then washed with a mixture of 2% FBS and PBS, followed by centrifugation at 1000 rpm for 5 minutes. The supernatant was discarded, and the cells were suspended in PBS, and 1×10⁵ cells for each sample were dispensed into a well plate. An antibody (R-phycoerythrin-conjugated mouse anti-human monoclonal antibody) was placed into each well and incubated on ice for 40 minutes. After the incubation, the medium was centrifuged at 1000 rpm for 5 minutes. The supernatant was removed and the cells were washed with PBS two times. After washing, the cells were fixed with 1% paraformaldehyde, thus analyzing surface antigens of multipotent stem cells using a flow cytometer (Table 1 and FIG. 2).

**Table 1: FACS analysis of surface antigens of adipose-derived stem cells**

| Surface antigens | AD-MSCs |
|---|---|
| CD73 | + |
| CD90 | + |
| CD29 | + |
| CD44 | + |
| CD105 | + |
| CD33 | - |
| CD34 | - |
| CD45 | - |
| CD4 | - |
| CD31 | - |
| CD62p | - |
| CD14 | - |
| HLA-DR | - |

As a result, as shown in Table 1, the adipose tissue-derived adult stem cells according to the present invention showed positive responses of 91 % to CD73, 97% to CD90, 96% to CD29, 83% to CD44, and 80% to CD105. Also, the inventive stem cells showed negative immunological responses to all of CD33, CD34, CD45, CD4, CD31, CD62p, CD14 and HLA-DR.

### Example 3: Formation of human adipose tissue-derived miltupotent stem cell spheres

Human adipose tissue-derived multipotent stem cells obtained in Example 1 were stored in conditions of saline, saline + sucrose, saline + sucrose + 5% albumin and PBS + sucrose, and then examined for their sphere formation ability.

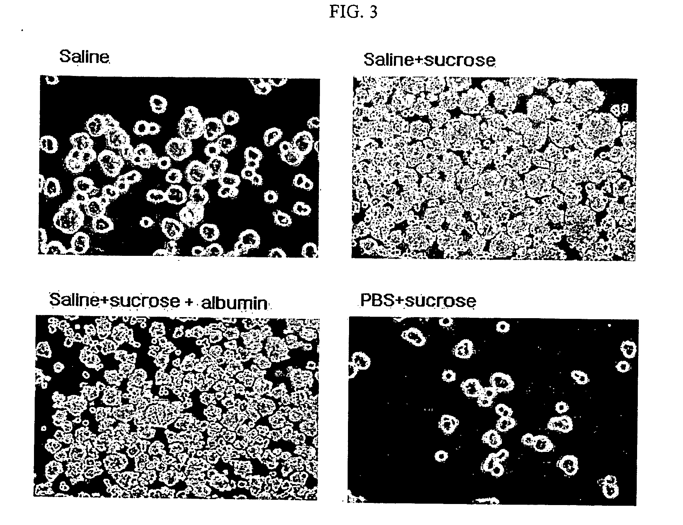

5×10⁴-1×10⁵/ml of the human adipose tissue-derived multipotent stem cells obtained in Example 1 were seeded into each well of a 6-well plate containing a serum-free MEBM medium supplemented with 10 µM CORM-2, 5 ml antibiotic antimycotic solution (100X), 1 µg/ml hydrocortisone, 5 µg/ml insulin, 20 ng/ml EGF, 40 ng/ml FGF, B27 and β-mercaptoethanol. As a result, the cells started to form the shape of spheres from 3-7 days after the seeding, and as shown in FIG. 3, the cells proliferated to form spheres even at 7-10 days after the seeding.

### Example 4: Examination of in vitro angiogenic effect of Adipose tissue-derived stem cells

Whether adipose tissue-derived mesenchymal stem cells form blood vessels was examined through an *in vitro* tube formation assay using cells, which were exposed or not exposed to a shear stress of 5 dynes/cm² (intravascular shear stress).

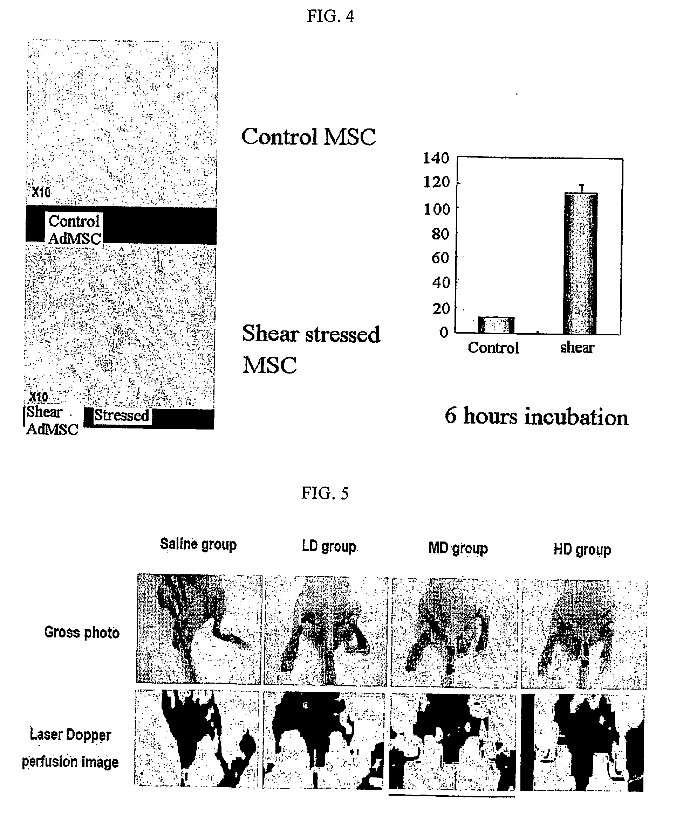

For this purpose, Matrigel (Chemicon, USA) was warmed and melted, and then the melted gel was added to a 96-well plate and solidified at room temperature for about 1 hour. Then, adipose tissue-derived multipotent stem cells obtained in Example 1 were dispensed in the well plate at a concentration of 1 x 10⁴ cells/well and incubated with EGM-2MV (Clonetics, USA) for 5 days, and whether the incubated cells would differentiate into vascular endothelial cells was examined. As a result, as shown in FIG. 4, it was observed, the inventive adipose tissue-derived mesenchymal stem cells, when exposed to shear stress, differentiated into vascular endothelial cells to form a structure similar to a vascular network.

### Example 5: Examination of in vivo angiogenic effect of adipose tissue-derived stem cells

### 5-1: Construction of mouse ischemic model

After 8-week-old nude mice were generally anesthetized, the midline of the left legs was incised, and the femoral arteries and their side branches were isolated and ligated. Then, the left femoral arteries were completely removed, thus creating an ischemic lower limb model.

### 5-2: Angiogenic effect of adipose tissue-derived stem cells in mouse ischemic model

At 24 hours after the mouse ischemic model constructed in Example 5-1 was created, the mouse models were divided, according to cell concentration, into three groups: an LD group (1×10⁶ cells/kg), an MD group (5×10⁶ cells/kg) and a HD group (1×10⁷ cells/kg). Then, the cells were injected intramuscularly into the ischemic sites of the mice.

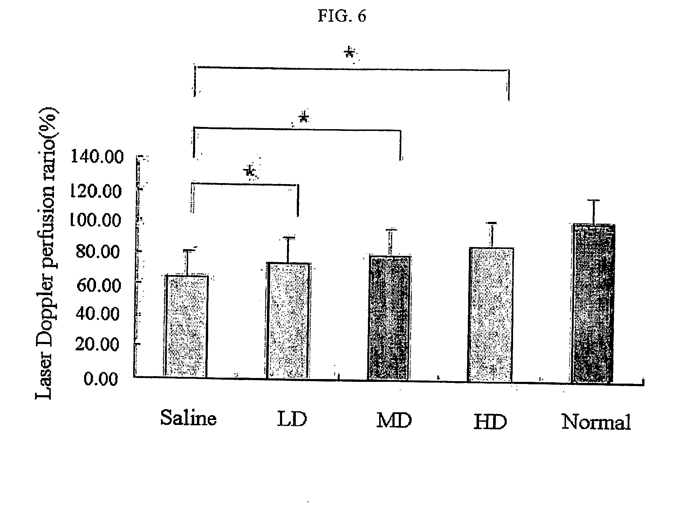

Whether lower limb removal would occur was continuously observed, the degree of improvement of blood flow in the mice was assessed through laser Doppler perfusion imaging at 4 weeks after the cell therapy. As a result, in the negative control group injected only with saline, lower limb removal occurred, and it could be observed in the laser Doppler perfusion images that the higher the concentration of the injected cells, the smoother the blood flow (FIG. 5). When the degree of improvement of blood flow was analyzed with respect to laser Doppler perfusion ratio, the group injected with the inventive adipose tissue-derived stem cells showed a dose-dependent improvement in blood flow compared to the saline-injected group as the control group (FIG. 6).

### Example 6: Formulation examples of stem cell therapeutic composition for cold storage

In formulations for cold storage at a temperature of about 4°C until they are administered by injection, the viabilities of cells according to the ratios of physiological saline, Hartman-D solution, PBS and sucrose as an isotonic solution useful to keep the stability of cells were examined. Also, to establish conditions in which cells are not aggregated or precipitated during cold storage, the effects of an increase and decrease in the contents of EDTA and human serum albumin on cell viabilities were examined (Table 2).

As a result, in comparison with the case where cells were stored in physiological saline, Hartman-D solution or PBS alone, in the case of formulations, comprising sucrose as an isotonic agent, albumin as an adsorption-preventing agent, or EDTA, a higher cell viability was shown, and the cells were not aggregated.

**Table 2: Cell viability in cold storage conditions**

| Cell viability (%) | 0 | 3 | 6 | 9 | 12 | 24 | 48 | 72 |
|---|---|---|---|---|---|---|---|---|
| Physiological saline | 97.1 | 96.3 | 93.2 | 90.9 | 89.2 | 86.9 | 70.3 | 57.2 |
| Physiological saline + 1% sucrose | 97.8 | 92.4 | 93.1 | 88.8 | 84.2 | 71.5 | 67.2 | 59.0 |
| Physiological saline + 2% sucrose | 97.9 | 92.2 | 89.4 | 89.6 | 79.9 | 77.4 | 78.9 | 64.9 |
| Physiological saline + 10% sucrose | 98.7 | 93.9 | 88.3 | 89.0 | 83.0 | 73.3 | 75.5 | 63.8 |
| Physiological saline + 2%sucrose + 2%albumin | 97.8 | 98.0 | 95.5 | 90.8 | 82.4 | 83.3 | 71.5 | 72.1 |
| Physiological saline + 2%sucrose + 5%albumin | 98.3 | 96.9 | 95.7 | 86.1 | 85.5 | 80.6 | 78.4 | 75.0 |
| **Physiological saline + 2%sucrose + 5%albumin + 1mM EDTA** | **99.0** | **95.5** | **94.3** | **92.1** | **93.0** | **81.7** | **83.3** | **79.7** |
| Physiological saline + 2%sucrose + 2%albumin + 10mM EDTA | 97.7 | 95.3 | 96.0 | 93.9 | 88.8 | 83.2 | 84.5 | 78.6 |
| PBS | 99.1 | 94.5 | 91.2 | 89.3 | 90.7 | 85.5 | 69.2 | 54.8 |
| PBS+2%sucrose | 97.6 | 90.2 | 89.5 | 79.4 | 82.1 | 83.7 | 72.0 | 65.2 |
| PBS + 2%sucrose + 5%albumin | 98.4 | 89.5 | 92.2 | 86.1 | 83.2 | 81.7 | 77.9 | 70.1 |
| Hartman-D | 99.4 | 90.0 | 91.5 | 82.2 | 81.9 | 70.2 | 55.3 | 54.9 |
| Hartman-D + 2%sucrose | 97.2 | 92.1 | 84.0 | 86.7 | 83.3 | 79.2 | 72.9 | 71.2 |
| Hartman-D + 2%sucrose + 5%albumin | 97.7 | 93.8 | 87.3 | 79.2 | 80.9 | 81.3 | 75.0 | 71.4 |

### Example 7: Formulation examples of stem cell therapeutic composition for frozen storage

Cell viability in various formulations comprising various additives including cryopreservatives, in addition to physiological saline as a base was examined in order to ensure the freeze-thaw stability of the inventive stem cell therapeutic composition during the frozen storage of the stem cell therapeutic composition.

Cell viability resulting from the addition of physiological saline, Hartman-D solution or PBS as an excipient, sucrose or mannose as an isotonic solution useful to keep the stability of cells, human serum albumin, which is used in cell cryopreservation, and DMSO, was observed after cell freezing and thawing.

As a result, it was observed that cell viability in frozen storage conditions was further increased in a formulation comprising physiological saline, PBS or Hartman-D solution, as a base, 2% sucrose and 5% albumin, and in a formulation comprising, in addition to these materials, cryopreservative DMSO (Table 3).

**Table 3: Cell viability in frozen storage conditions**

| Cell viability (%) | 0 | 24h | 48h | 72h |
|---|---|---|---|---|
| Physiological saline | 97.1 | 82.3 | 80.0 | 81.3 |
| Physiological saline + 2% sucrose | 97.9 | 78.9 | 81.3 | 82.2 |
| Physiological saline + 10%DMSO | 96.6 | 87.5 | 86.5 | 80.9 |
| **Physiological saline + 2% sucrose + 5%albumin** | **98.3** | **80.4** | **84.2** | **83.1** |
| **Physiological saline + 2% sucrose + 5%albumin + 10%DMSO** | **97.7** | **89.7** | **87.9** | **88.0** |
| Physiological saline + 2% mannose | 98.1 | 83.0 | 80.1 | 79.8 |
| Physiological saline + 2% mannose + 5%albumin | 97.3 | 87.7 | 83.4 | 82.1 |
| Physiological saline + 2% mannose + 5%albumin + 10%DMSO | 98.5 | 86.4 | 87.3 | 87.9 |
| PBS | 99.1 | 82.3 | 79.0 | 77.4 |
| PBS + 2%sucrose | 97.2 | 87.2 | 78.0 | 83.5 |
| PBS + 10%DMSO | 97.6 | 84.8 | 86.5 | 82.3 |
| PBS + 2%sucrose + 5%albumin | 98.4 | 83.2 | 81.8 | 81.0 |
| PBS + 2%sucrose + 5%albumin + 10%DMSO | 98.0 | 86.2 | 79.3 | 85.4 |
| Hartman-D | 99.4 | 73.6 | 69.9 | 65.4 |
| Hartman-D + 2%sucrose | 97.2 | 70.2 | 78.9 | 73.2 |
| Hartman-D + 10%DMSO | 98.0 | 84.2 | 83.7 | 81.1 |
| Hartman-D + 2%sucrose + 5%albumin | 97.7 | 87.1 | 76.6 | 79.3 |
| Hartman-D + 2%sucrose + 5%albumin + 10%DMSO | 96.9 | 88.0 | 83.2 | 87.3 |

### INDUSTRIAL APPLICABILITY

As described in detail above, the present invention provides cell therapeutic compositions and their use for treating ischemic diseases, wherein the compositions contain human adipose tissue-derived mesenchymal stem cells as active ingredients. The cell therapeutic compositions according to the present invention induce angiogenesis around closed blood vessels in the ischemic lesions to boost blood flow, and thus are useful to treat ischemic diseases.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A cell therapeutic composition, which contains 1 x 10⁵ - 1 x 10⁸ cells/mL adipose tissue-derived mesenchymal stem cells, physiological saline, 2% sucrose, 5% albumin and 1mM EDTA.

2. A cell therapeutic composition, which contains 1 x 10⁵ - 1 x 10⁸ cells/mL adipose tissue-derived mesenchymal stem cells, physiological saline, 2% sucrose, 2% albumin and 10mM EDTA.

3. A cell therapeutic composition, which contains 1 x 10⁵ - 1 x 10⁸ cells/mL adipose tissue-derived mesenchymal stem cells, physiological saline, 2% sucrose, 5% albumin and 10% DMSO.

4. The cell therapeutic composition of any one of claims 1 to 3 for use for treating ischemic diseases.

## Patentansprüche

1. Zelltherapeutische Zusammensetzung, die 1 x 10⁵ - 1 x 10⁸ Zellen/ml von Fettgewebe stammende mesenchymale Stammzellen, physiologische Kochsalzlösung, 2% Saccharose, 5% Albumin und 1 mM EDTA enthält.

2. Zelltherapeutische Zusammensetzung, die 1 x 10⁵ - 1 x 10⁸ Zellen/ml von Fettgewebe stammende mesenchymale Stammzellen, physiologische Kochsalzlösung, 2% Saccharose, 2% Albumin und 10 mM EDTA enthält.

3. Zelltherapeutische Zusammensetzung, die 1 x 10⁵ - 1 x 10⁸ Zellen/ml von Fettgewebe stammende mesenchymale Stammzellen, physiologische Kochsalzlösung, 2% Saccharose, 5% Albumin und 10% DMSO enthält.

4. Zelltherapeutische Zusammensetzung gemäß einem der Ansprüche 1 bis 3 zur Verwendung für die Behandlung von ischämischen Erkrankungen.

## Revendications

1. Composition thérapeutique de cellules, contenant 1x10⁵ - 1x10⁸ cellules/ml des cellules souches mésenchymateuses dérivées de tissu adipeux, de la solution saline physiologique, 2% de saccharose, 5% d'albumine et 1mM EDTA.

2. Composition thérapeutique de cellules, contenant 1x10⁵ - 1x10⁸ cellules/ml des cellules souches mésenchymateuses dérivées de tissu adipeux, de la solution saline physiologique, 2% de saccharose, 2% d'albumine et 10mM EDTA.

3. Composition thérapeutique de cellules, contenant 1x10⁵ - 1x10⁸ cellules/ml des cellules souches mésenchymateuses dérivées de tissu adipeux, de la solution saline physiologique, 2% de saccharose, 5% d'albumine et 10% de DMSO.

4. Composition thérapeutique de cellules selon l'une quelconque des revendications 1 à 3 pour l'utilisation pour traiter des maladies ischémiques.
